# EUROPEAN PATENT APPLICATION

(11) **EP 1 344 529 A1**
(43) Date of publication of application: **17.09.2003**
(21) Application number: 03251525.6
(22) Date of filing: 13.03.2003
(51) Int. Cl.: A61K 35/78, A61P 5/26, A61P 17/14

(54) **Therapeutic compositions for treating male pattern baldness**

(30) Priority: 13.03.2002 GB 0205826
(71) Applicant: Bryant, Andrew Edward, Aldershot, Hampshire GU12 5SF (GB)
(72) Inventor: Bryant, Andrew Edward, Aldershot, Hampshire GU12 5SF (GB)
(74) Representative: Brown, Michael Stanley

(57) **Abstract**

A therapeutic formulation for use in the treatment of male pattern baldness includes:-
a) an ingredient which inhibits aromatase activity, i.e. reduces the rate at which testosterone is converted to oestradiol, and
b) an ingredient which reduces the amount of testosterone which is bound to the SHGB, and/or
c) an ingredient which increases the level of testosterone.

## Description

### Field of the Invention

This invention relates to therapeutic compositions and methods and, while the invention has been developed in relation to the treatment of male pattern baldness, the therapeutic compositions and methods which have been developed have other applications, as explained below.

### Background to the Invention

Male pattern baldness increases in men as they age and is believed to be related to the presence of the potent hormone dihydrotestosterone in the male body as a result of conversion of the male hormone testosterone. Such conversion is made possible by enzymes known as 5-alpha-reductase.

Chemically or physically castrating a male dramatically lowers the amount of testosterone available in the male body. Such reduction in the amount of testosterone, i.e. a reduction in the amount of raw material available for conversion, produces an equally dramatic reduction in the amount of dihydrotestosterone which is made. Most men would, of course, not opt for such a drastic measure and, as a result, research efforts relating to the treatment of male pattern baldness have been directed towards inhibition of the process by which testosterone is converted to dihydrotestosterone.

Testosterone is transported around the human body on a carrier molecule known as Sex Hormone Binding Globulin (SHBG). It is only the portion of free unbound testosterone which is active and able to create androgenic effects in the body.

As a male ages, there is a reduction in the level of free testosterone in the body and part of this reduction results from an increase in the level of SHBG and hence an increase in the proportion of the available testosterone which is bound to the carrier molecule.

In addition, as a male ages, luteinising hormone and follicle stimulation hormone increase, thereby providing an indication of a reduction in the level of testosterone.

Current treatment procedures for male pattern baldness have concentrated on inhibition of the conversion of testosterone to dihydrotestosterone but have ignored the fact that the level of the basic raw material needed to make dihydrotestosterone is decreasing and hence indicating that the body is up-regulating the overall amount of dihydrotestosterone it is able to yield from its testosterone.

It is an object of the present invention to provide a new and improved method of treating male pattern baldness.

It is another object of the present invention to provide a new and improved therapeutic composition and a new and improved therapeutic method.

### Summary of the Invention

According to a first aspect of the present invention there is provided a method of treating male pattern baldness, i.e. a method of reducing hair loss, said method comprising the administration of a formulation which includes an ingredient which inhibits aromatase activity, i.e. reduces the rate at which testosterone is converted to oestradiol and, at the same time, increasing the level of testosterone.

The ingredient which inhibits aromatase activity preferably is or includes a plant or herb extract. It may alternatively be a drug.

Increasing the level of testosterone may be effected by means of testosterone patches or injections or by means of sustained release pellets containing testosterone.

The method may also include administering an ingredient which reduces the amount of testosterone which is bound to the SHGB.

The ingredient which reduces the amount of testosterone which is bound to the SHGB preferably is or includes a plant or herb extract or a drug having a similar structure to testosterone such that it will bind to the SHGB in place of some of the testosterone.

In addition, the method may include the use of plant or herb extracts which improve the P450 function of the liver and thereby remove excessive oestradiol and dihydrotestosterone.

At the same time, if required, plant or herb extracts or drugs may be used which inhibit the production of dihydrotestosterone, and thereby have a more rapid effect on the reversal of hair loss.

According to a second aspect of the present invention there is provided a therapeutic formulation which includes:-
a) an ingredient which inhibits aromatase activity, i.e. reduces the rate at which testosterone is converted to oestradiol, and
b) an ingredient which reduces the amount of testosterone which is bound to the SHGB, and/or
c) an ingredient which increases the level of testosterone.

As mentioned above, the ingredient which inhibits aromatase activity preferably is or includes a plant or herb extract. In addition, the ingredient which reduces the amount of testosterone which is bound to the SHGB preferably is or includes a plant or herb extract or a drug having a similar structure to testosterone such that it will bind to the SHGB in place of some of the testosterone.

When obtaining the extracts, it is possible to use the whole plant or part thereof. Standardised extracts of different strengths may also be used.

The formulation may also include one or more vitamins or minerals to increase the efficiency of the plant or herb extract(s). Suitable vitamins include Vitamin B3, Vitamin B6 and Vitamin B5 (pantothenic acid). Suitable minerals include chromium and zinc salts.

The formulation may be in the form of a tablet, capsule, powder, topical lotion or cream, or a sub-lingual spray, liquid or powder.

There is a balance between the amount of free unbound testosterone in the body of a male and the amount of oestradiol which is present. This balance can vary and the zone in which the two opposing hormones can vary is predetermined genetically. Once the oestradiol levels begin to rise beyond the desired zone, the body of a male reacts by increasing the amount of dihydrotestoserone which is produced, as this is a more potent androgen than testosterone and increasing the amount of dihydrotestosterone is a more rapid way of restoring the required masculine balance.

Some males may have an apparently normal testosterone level, but this can be converted to oestradiol by the enzyme aromatase. Increasing the testosterone level in such cases will then produce an increase in the level of oestradiol, with a consequent increase in the level of dihydrotestosterone.

An increase in the level of oestradiol produces an increase in the level of SHBG, and this increases the amount of testosterone which is bound to the carrier molecule and further reduces the level of free testosterone.

Increased levels of oestradiol can also produce increased fat storage in a male and the fat cells can themselves produce aromatase, the presence of which will result in an increase in the rate of conversion of testosterone to oestradiol. A "vicious circle" can thus be set up, with an increase in fat storage producing an increase in oestradiol levels, which further increases the fat storage and increasingly upsets the balance between the male and female hormones.

Use of the formulation of the present invention enable this "vicious circle" to be avoided.

In addition to the use of the formulation for the treatment of male pattern baldness, the formulation has other uses. Thus, it may be used for treating and/or reducing the incidence of prostate cancer. It may also be used for increasing the sex drive of males and for improving muscle definition and for reducing the incidence of heart disease. In addition, the formulation may be used as part of a male body building programme. Thus changing the balance of the hormones will assist in producing increased muscle mass whilst reducing body fat.

The formulation may also be used for reducing the growth of unwanted facial hair, for example, reducing the growth of hair in the nostrils and ears of a male, reducing excessive eyebrow growth and reducing the rate of growth of beard stubble and the diameter of the facial hairs.

The active ingredient which inhibits aromatase activity may be Oenothein A and/or B, which are found in the following species:-
a) Epilobium Angustifolium,
b) Epilobium Capense,
c) Epilobium Dodonaei,
d) Epilobium Hirsutum,
e) Epilobium Montanum,
f) Epilobium Parviflorum,
g) Epilobium Roseum,
h) Epilobium Salignum, and
i) Epilobium Stereophyllum.

The active ingredient which inhibits aromatase activity may alternatively, or additionally, be the anti-oxidant Chrysin.

The ingredient which raises the level of free testosterone may be or include Muira Puama, Tribulus Terrestris, nettle extract, Eurycome longifolia jack and/or Avena Sativa.

The formulation may also include Resveratrol (which is extracted from red grapes) to remove excessive levels of hormones from the liver by improving the P450 function of the liver.

### Description of the Preferred Embodiment

In one example of the present invention, a user is provided with two liquid formulations, i.e. Formulation A and Formulation B, each of which contains active ingredients and the person being treated for male pattern baldness will normally take a caplet of each formulation per day.

A caplet of Formulation A contains the following active ingredients:
a) pantothenic acid - as d-calcium pantothenate - 100 mg.,
b) chromium - as chromium picolinate - 60 mcg., and
c) epilobium- 250 mg.

Formulation A also contains the following additional ingredients:
a) modified cellulose gum,
b) silica,
c) di-calcium phosphate,
d) magnesium stearate, and
e) stearic acid.

A caplet of formulation B contains the following active ingredients:
a) niacin- 20 mg.,
b) Vitamin B6 - as Pyroxidine HCI- 10 mg.,
c) zinc - as zinc citrate - 30 mg.,
d) L-Argine - 100 mg.,
e) Tribulus terretris 40% strength - 200 mg.,
f) Muira puama 10:1 extract - 20 mg., (equivalent to 200 mg.,)
g) Avena sativa 10:1 extract - 20 mg., (equivalent to 200 mg.,) and
h) nettle root - 200 mg.

Formulation B also includes the same additional ingredients that have been mentioned above with reference to Formulation A.

Some users may experience a transient flushing and feeling of warmth associated with the ingestion of products containing niacin. It is accordingly possible to vary the constituents of each formulation as appropriate. It is also possible to vary the relative volumes of each formulation taken by a user each day.

It is also possible to produce tablets containing the active ingredients included in Formulations A and B in the relative proportions indicated above, or such variations thereof that will be most effective (or appropriate) for a particular user.

## Claims

1. A method of treating male pattern baldness, i.e. a method of reducing hair loss, said method comprising the administration of a formulation which includes an ingredient which inhibits aromatase activity, i.e. reduces the rate at which testosterone is converted to oestradiol and, at the same time, increasing the level of testosterone.

2. A method as claimed in Claim 1, in which the ingredient that inhibits aromatase activity is or includes a plant extract, e.g. herb extract.

3. A method as claimed in either of the preceding claims, in which increasing the level of testosterone is effected by means of testosterone patches.

4. A method as claimed in Claim 1 or Claim 2, in which increasing the level of testosterone is effected either by injections or by means of sustained release pellets containing testosterone.

5. A method as claimed in any one of the preceding claims, which includes administering an ingredient that reduces the amount of testosterone that is bound to the SHBG (Sex Hormone Binding Globulin).

6. A method as claimed in Claim 5, in which the ingredient is or includes a plant or herb extract or a drug having a similar structure to testosterone such that it will bind to the SHGB in place of some of the testosterone.

7. A method as claimed in any one of the preceding claims, which additionally includes the use of plant or herb extracts or drugs that:-
a) improve the P450 function of the liver and thereby remove excessive oestradiol and dihydrotestosterone and/or
b) inhibit the production of dihydrotestosterone and thereby have a more rapid effect on the reversal of hair loss.

8. A therapeutic formulation which includes:-
a) an ingredient which inhibits aromatase activity, i.e. reduces the rate at which testosterone is converted to oestradiol, and
b) an ingredient which reduces the amount of testosterone which is bound to the SHGB, and/or
c) an ingredient which increases the level of testosterone.

9. A therapeutic formulation as claimed in Claim 8, in which the ingredient that inhibits aromatase activity is or includes a plant or herb extract.

10. A therapeutic formulation as claimed in Claim 8 or 9, in which the ingredient which reduces the amount of testosterone which is bound to the SHGB is or includes a plant or herb extract or a drug having a similar structure to testosterone such that it will bind to the SHGB in place of some of the testosterone.

11. A therapeutic formulation as claimed in any one of Claims 8 to 10, in which the active ingredient that inhibits aromatase activity is Oenothein A and/or B.

12. A therapeutic formulation as claimed in any one of Claims 8 to 10, in which the active ingredient that inhibits aromatase activity is the anti-oxidant Chrysin.

13. A therapeutic formulation as claimed in any one of Claims 8 to 12, in which the ingredient which raises the level of free testosterone is or includes Muira Puama, Tribulas Terrestris, nettle extract, Eurycoma longifolia jack and/or Avena Sativa.

14. A therapeutic formulation as claimed in any one of Claims 8 to 13, which also includes Resveratrol.
